# EUROPÄISCHE PATENTSCHRIFT

(11) **EP 1 622 583 B1**
(45) Veröffentlichungstag und Bekanntmachung des Hinweises auf die Patenterteilung: **21.12.2016**
(21) Anmeldenummer: 04731350.7
(22) Anmeldetag: 06.05.2004
(51) Int. Cl.: A61Q 5/12, A61K 8/81, A61Q 5/06

(54) **HAARBEHANDLUNGSMITTEL MIT STYLING-EIGENSCHAFTEN**
HAIR CARE PRODUCTS WITH STYLING PROPERTIES
PRODUITS DE TRAITEMENT CAPILLAIRE A PROPRIETES COIFFANTES

(30) Priorität: 15.05.2003 DE 10322059
(43) Veröffentlichungstag der Anmeldung: 08.02.2006
(73) Patentinhaber: Henkel AG & Co. KGaA, 40589 Düsseldorf (DE)
(72) Erfinder: ZÜDEL FERNANDES, Nicole, 20459 Hamburg (DE); RICHTERS, Bernd, 21129 Hamburg (DE); SCHULZE ZUR WIESCHE, Erik, 20251 Hamburg (DE)
(86) Internationale Anmeldenummer: PCT/EP2004/004825
(87) Internationale Veröffentlichungsnummer: WO 2004/100908

(56) Entgegenhaltungen:
- WO-A-01/74310
- WO-A-01/76543
- WO-A-01/91705
- WO-A-02/096381

## Beschreibung

Die Erfindung betrifft Haarbehandlungsmittel mit einer speziellen Polymerkombination aus einem verdickenden Polymer und einem Acrylterpolymer, die Verwendung der Haarbehandlungsmittel zur Fixierung und Pflege von Haaren sowie ein Verfahren zur Behandlung von Haaren mit diesen Mitteln.

Keratinische Fasern, insbesondere menschliche Haare, werden heutzutage einer Vielzahl von Behandlungen unterzogen. Dabei spielen Behandlungen, die zu einer permanenten oder temporären Formgebung der Haare dienen, eine wichtige Rolle. Temporäre Formgebungen, die einen guten Halt ergeben sollen, ohne dass das gesunde Aussehen der Haare, wie zum Beispiel deren Glanz, beeinträchtigt wird, können beispielsweise durch Haarsprays, Haarwachse, Sprühfestiger, Fönwellen, Schäume, Haargele etc. erzielt werden.

Üblicherweise werden Haarsprays und Sprühfestiger als einphasige Produkte in blickdichten Verpackungen angeboten. Es hat sich in der letzten Zeit jedoch gezeigt, dass der Verbraucher neben den Ansprüchen an den Halt und die Pflege auch immer höhere Anforderungen an das Design und die einfache Handhabbarkeit der Produkte stellt, so dass nun verstärkt Produkte in transparenten Verpackungen hergestellt werden, die dem Verbraucher die Erkennbarkeit des Füllstandes erleichtern und den Produkten eine größere Attraktivität verleihen.

Es sind daher bereits Produkte bekannt, die ein-, zwei- oder dreiphasig formuliert und je Phase unterschiedlich eingefärbt sein können.

Weiterhin kennt man klare Produkte, in die farbige Pigmentteilchen eingearbeitet werden können, die in dem Produkt schweben.

Die Schwierigkeit bei der Herstellung solcher Produkte war bislang, dass die Viskosität der Produkte ausreichend hoch sein muss, damit die Pigmentteilchen stabil in der Schwebe gehalten werden können. Hochviskose Produkte lassen sich aber nur schwer oder gar nicht versprühen, was sie für die Anwendung als Haarspray oder Sprühfestiger unbrauchbar macht. Bei geringerer Viskosität beobachtete man jedoch bisher ein Absetzen der Pigmentteilchen, was die Produkte unattraktiv und für den Verbraucher uninteressant macht.

Aus der EP 256 427 B1 sind Pigmentdispersionen bekannt, die sich für die Verwendung in kosmetischen Produkten eignen und neben dem Pigment ein Alkylglycolethersulfat und einen Phosphorsäureester als zwingende Komponenten für eine stabile Dispergierung der Pigmente, enthalten.

In der WO 99/01512 A1 werden stabile, spezielle Pigmentdispersionen offenbart, die ein Polymer, ein Pigment, ein wasserhaltiges und ein nicht-wasserhaltiges Lösungsmittel enthalten, um eine stabile Dispergierung zu erreichen.

Neben der Stabilisierung von Pigmenten war bisher aber auch die Einarbeitung kationischer Pflegestoffe in versprühbare Haarbehandlungsmittel mit ausreichend niedriger Viskosität problematisch, weshalb ein weiteres Ziel war Mittel zu entwickeln, die trotz niedriger Viskosität die stabile Einarbeitung kationischer Pflegestoffe gewährleisten.

Vollkommen überraschend konnte nun ein Haarbehandlungsmittel hergestellt werden, das durch die Kombination zweier spezieller Polymere die oben genannten Anforderungen in hohem Maße erfüllt. Durch die Kombination der Polymere gelangt man zu klaren, flüssigen Formulierungen, die in der Lage sind trotz der geringen Viskosität Pigmente in Schwebe zu halten und gleichzeitig die Einarbeitung kationischer Pflegestoffe zu erlauben. Demzufolge erreicht man haarkonditionierende Effekte neben hervorragenden Halteffekten am Haar, ohne dass den Mitteln zwangsläufig noch ein haarfixierendes Polymer zugesetzt werden muss.

Gegenstand der Erfindung sind daher Haarbehandlungsmittel, enthaltend in einem kosmetisch akzeptablen Träger
a) 0,001 bis 15 Gew.-% mindestens eines von b) verschiedenen, verdickend wirkenden Polymers und
b) 0,001 bis 15 Gew.-% mindestens eines Acrylterpolymers, das zusammengesetzt ist aus
   - 5 bis 80% eines Acrylmonomers A, das ausgewählt ist aus C₁-C₆-Alkylacrylaten und C₁-C₆-Alkylmethacrylaten und
   - 5 bis 80% eines Monomers B, das ausgewählt ist aus Vinyl-substituierten Heterocyclen, die mindestens ein Stickstoff-oder Schwefelatom enthalten, (Meth)acrylamid, Mono- oder Di-C₁₋₄-alkyl(meth)arcylaten und Mono- oder Di-C₁₋₄-alkylamino-C₁₋₄-alkyl-(meth)-acrylamiden, und
   - 0,1 bis 30% eines Monomers C, das ausgewählt ist aus
      - einem ethylenisch ungesättigten copolymerisierbaren oberflächenaktiven Monomer, erhältlich durch Kondensation eines nichtionischen Tensids mit einer α,β-ethylenisch ungesättigten Carbonsäure oder dem Anhydrid davon und
      - einem oberflächenaktiven Monomer, ausgewählt aus dem Harnstoff-Reaktionsprodukt eines monoethylenisch ungesättigten Monoisocyanats mit einem nicht-ionischen Tensid mit Amin-Funktionalität,
dadurch gekennzeichnet dass es eine Viskosität von 100 Bis 600 mPas aufweist, gemessen bei 20°C mit Brookfield RVDVII+Viskosimeter bei 20 UpH, Spindel 3.

Geeignete erfindungsgemäße verdickende Polymere a) umfassen mindestens ein Monomer auf Acrylat- oder Methacrylatderivatbasis. Solche verdickenden Polymere a) sind ausgewählt aus Homopolymeren der Formel (I), 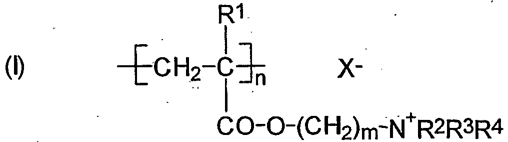 in der R¹ für Wasserstoff oder eine Methylgruppe steht, R², R³ und R⁴ unabhängig voneinander für C₁₋₄-Alkyl-, -Alkenyl- oder -Hydroxyalkylgruppen stehen; m für Zahlen von 1 bis 4 steht, n für eine natürliche Zahl und X- für ein physiologisch verträgliches organisches oder anorganisches Anion steht, sowie Copolymeren, bestehend im wesentlichen aus den in Formel (I) aufgeführten Monomereneinheiten sowie nichtionogenen Monomereneinheiten.

Die Eigenschaft "verdickend wirkend" des Polymers a) ist im Rahmen der Erfindung wie folgt definiert: die Zugabe von 0,5 Gewichtsteilen des Polymers zu 100 Gewichtsteilen entsalztem Wasser bewirkt eine Erhöhung der Viskosität auf den mindestens 50-fachen Wert, bezogen auf die Viskosität des entsalzten Wassers. Die Bestimmung der Viskosität wird bei 20°C mit einem Viskosimeter der Marke Brookfield RVDVII+ durchgeführt (Speed: 20 Umdrehungen pro Minute; Spindel 1 bei Viskositäten bis zu 200 m*Pas, Spindel 3 bei Viskositäten von 200-2000 m*Pas oder Spindel 5 bei Viskositäten über 2000 m*Pas; Ablesen des Messwerts jeweils 20 Sekunden nach Beginn der Messung).

Polymere a), die eine Erhöhung der Viskosität auf mindestens den 100-fachen Wert bewirken, sind bevorzugt.

Bevorzugte Haarbehandlungsmittel im Sinne der Erfindung enthalten Homopolymere nach Formel (I) oder Copolymere aus Monomereneiheiten der Formel (I) und nichtionogenen Monomereneinheiten, in denen die Reste R¹ bis R⁴ für Methylgruppen stehen und m den Wert 2 hat.

Als physiologisch verträgliches Gegenion X- kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Gemäß einer ersten, besonders bevorzugten Ausführungsform der Erfindung werden als verdickende Polymere a) Homopolymere nach Formel (I) eingesetzt. Im Rahmen dieser Ausführungsform bevorzugt ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabinol, Mannitol, Sorbitol, Sucrose oder Glucose erfolgen. Methylenbisacrylamid ist ein bevorzugtes Vernetzungsagens.

Ein erfindungsgemäß besonders geeignetes Homopolymer ist im Handel beispielsweise unter der Bezeichnung Synthalen^{®} CR von der Firma 3V Sigma oder unter der Bezeichnung Rheocare^{®} CTH oder Rheocare^{®} Ultragel 300 von der Firma Cosmetic Rheologies Ltd. erhältlich.

Es kann erfindungsgemäß bevorzugt sein, das Homopolymer in Form einer nichtwässrigen Polymerdispersion, die einen Polymeranteil nicht unter 30 Gew.-% aufweisen sollte, einzusetzen. Solche Polymerdispersionen sind unter den Bezeichnungen Salcare^{®} SC 95 (ca. 50% Polymeranteil; weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50% Polymeranteil; weitere Komponenten: Mischung von Diestern des Propylenglycols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Gemäß einer zweiten, bevorzugten Ausführungsform der Erfindung werden als verdickende Polymere a) Copolymere eingesetzt, deren nichtionogene Monomereneinheiten ausgewählt sind aus der Gruppe, die von Acrylamid; Methacrylamid, Acrylsäure-C₁₋₄-Alkylestern, Methacrylsäure-C₁₋₄-Alkylestern und Vinylpyrrolidon gebildet wird.

Insbesondere bevorzugt sind verdickende Polymere a), die Copolymere aus Monomereneinheiten der Formel (I) und Acrylamid und Copolymeren aus Monomereneinheiten der Formel (I) und Vinylpyrrolidon sind.

Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomeren in einem Gewichtsverhältnis von 20 : 80 vorliegen, sind im Handel als ca. 50%ige nichtwässrige Polymerdispersion unter der Bezeichnung Salcare^{®} SC 92 erhältlich.

Weitere Informationen über die Produkte Salcare^{®} SC 95, Salcare^{®} SC 96 und Salcare^{®} SC 92 sind den Patentschriften EP-B1-686 024 und EP-B1-761 206, ferner den Druckschriften "Richtrezepturen Salcare^{®} SC 95", "Richtrezepturen Salcare^{®} SC 96" sowie "Salcare^{®} SC 95 (TPD 6237)" und Salcare^{®} SC 96 (TPD 6252)" der Allied Colloids GmbH zu entnehmen.

Ein bevorzugtes Copolymer aus Monomereneinheiten der Formel (I) und Vinylpyrrolidon wird im Handel unter der Handelsbezeichnung Aristoflex^{®} AVC von der Firma Clariant vertrieben.

Die verdickenden Polymere a) werden in den erfindungsgemäßen Haarbehandlungsmitteln üblicherweise in einer Menge von 0,001 bis 15 Gew.-%, bezogen auf das Gesamtgewicht des Mittels, eingesetzt. Bevorzugt ist eine Einsatzmenge der Polymere a) von 0,01 bis 5 Gew.-%, insbesondere bevorzugt von 0,05 bis 4 Gew.-%.

Bevorzugte Terpolymere b) im Sinne der Erfindung sind solche, bei denen das Monomer A ein C₂₋₆-Alkylacrylat ist; ganz besonders bevorzugt ist Ethylacrylat.

Bevorzugte Terpolymere b) im Sinne der Erfindung sind auch solche, bei denen das Monomer B ausgewählt ist aus N,N-Dimethylaminoethylmethacrylat, N,N-Diethylaminoethacrylat, N,N-Diethylaminoethylmethacrylat, N,t-Butylaminoethylacrylat, N,t-Butylaminoethylmethacrylat, N,N-Dimethylaminopropylacrylamid, N,N-Dimethylaminopropylmethacrylamid, N,N-Diethylaminopropylacrylamid, N,N-Diethylaminopropylmethacrylamid. Ein ganz besonders bevorzugtes Monomer B ist das N,N-Dimethylaminoethylmethacrylat.

Bevorzugte Terpolymere b) im Sinne der Erfindung sind solche, bei denen das Monomer C ein copolymerisierbares, ethylenisch ungesättigtes Tensidmonomer ist, das durch Kondensation eines nichtionischen grenzflächenaktiven Stoffes mit Itaconsäure entsteht.

Insbesondere bevorzugte Acrylterpolymere b) im Sinne der Erfindung sind solche, die aus Acrylaten, Amino(meth)acrylaten und mit 20 Mol Ethylenoxid polyethoxylierten C₁₀₋₃₀-Alkylitaconat zusammengesetzt sind. Diverse Herstellungsverfahren für die erfindungsgemäßen Acrylterpolymere sind dem Fachmann aus der EP-A-824 914 bekannt. Solche Acrylterpolymere sind im Handel beispielsweise unter dem Namen Structure^{®} Plus von der Firma National Starch als ca. 20%ige, wässrige Dispersion erhältlich.

Die Acrylterpolymere b) werden in den erfindungsgemäßen Haarbehandlungsmitteln üblicherweise in einer Menge von 0,001 bis 15 Gew.-%, bezogen auf das Gesamtgewicht des Mittels, eingesetzt. Bevorzugt ist eine Einsatzmenge der Polymere b) von 0,01 bis 10 Gew.-%, insbesondere bevorzugt von 0,05 bis 7 Gew.-%.

Für einige Anwendungen kann es vorteilhaft sein, dass die Acrylterpolymere mit 0,01 bis 8 Gew.-%, inbesondere mit 4 bis 8 Gew.-%, in den erfindungsgemäßen Haarbehandlungsmitteln eingesetzt werden.

In einer weiteren, bevorzugten Ausführungsform der Erfindung werden die Polymere a) und b) in einem Verhältnis a) : b) von 0,5 : 25, insbesondere im Verhältnis 1 : 16 eingesetzt.

In einer dritten, besonders bevorzugten Ausführungsform der Erfindung, handelt es sich bei den erfindungsgemäßen Haarbehandlungsmitteln um sprühbare, klare Formulierungen, in denen die Pigmente schweben.

Sprühbare Formulierungen weisen üblicherweise eine niedrige Viskosität auf; bevorzugt sollte die Viskosität der Formulierungen im Bereich von 50 bis 1500 mPa*s liegen. Besonders bevorzugt ist ein Viskositätsbereich der Haarbehandlungsmittel im Bereich von 100 bis 600 mPa*s. Die Viskosität der Mittel wurde bei 20°C mit einem Brookfield RVDVII+-Viskosimeter bei 20 UpM bestimmt. Für die Viskositätswerte bis 1000 mPa*s wurde die Spindel 3 und bei Viskositätswerte ab 1000 mPa*s wurde die Spindel 4 verwendet. Die Meßwerte wurden nach 20 Sekunden abgelesen.

In einer vierten, besonders bevorzugten Ausführungsform der erfindungsgemäßen Haarbehandlungsmittel kann die pflegende Wirkung durch die Verwendung von Proteinhydrolysaten und deren Derivaten weiter gesteigert werden. Proteinhydrolysate sind Produktgemische, die durch sauer, basisch oder enzymatisch katalysierten Abbau von Proteinen (Eiweißen) erhalten werden.

Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana, Seiwa), Sericin^{®} (Pentapharm), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Besonders bevorzugt im Sinne der Erfindung sind Seidenproteinhydrolysate, wie sie unter den Handelsbezeichnungen Promois^{®} Silk und Sericin^{®} im Handel erhältlich sind.

Erfindungsgemäß bevorzugt ist auch die Verwendung von Proteinhydrolysaten pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Besonders bevorzugte Proteine pflanzlichen Ursprungs im Sinne der Erfindung sind Weizenproteinhydrolysate, wie sie beispielsweise unter der Handelsbezeichnung Gluadin^{®} W 20 (INCI-Bezeichnung: Hydrolyzed Wheat Protein; ca. 20%ige wässrige Lösung) von der Firma Cognis im Handel erhältlich sind.

Wenngleich der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin oder Pyrroglutaminsäure eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Gluadin^{®} (Cognis), Lexein^{®} (Inolex), Crolastin^{®} (Croda) oder Crotein^{®} (Croda) vertrieben.

Erfindungsgemäß einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrotysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Darüber hinaus können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxipropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Prötein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein. Ganz besonders bevorzugt sind die kationischen Proteinhydrolysate und -derivate auf pflanzlicher Basis.

Besonders bevorzugte kationisierte Proteinhydrolysate pflanzlichen Ursprungs im Sinne der Erfindung sind kationisierte Weizenporteinhydrolysate, wie sie unter dem Handelsnamen Gluadin^{®} WQ (INCI-Bezeichnung: Laurdimonium Hydroxypropyl Hydrolyzed Wheat Protein; ca. 31-35%ige wässrige Lösung) von der Firma Cognis im Handel erhältlich sind.

Die Proteinhydrolysate sowie die kationisierten Proteinhydrolysate werden erfindungsgemäß bevorzugt in einer Menge von 0,1 bis 6 Gew.-%, insbesondere in einer Menge von 0,2 bis 2 Gew.-%, jeweils bezogen auf das Gesamtgewicht der Haarbehandlungsmittel, eingesetzt.

In einer fünften, besonders bevorzugten Ausführungsform der erfindungsgemäßen Haarbehandlungsmittel kann deren pflegende Wirkung durch die Verwendung von kationischen Tensiden und/oder kationischen Polymeren noch weiter gesteigert werden.

Beispiele für erfindungsgemäß geeignete kationische Polymere sind kationische Cellulosederivate, wie z.B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinyl-imidazol-Polymere, wie z. B. Luviquat^{®} (BASF), Kondensationsprodukte von Polyglycolen und Aminen, quaternierte Kollagenpolypeptide, wie beispielsweise Lauryldimonium hydroxypropyl hydrolyzed collagen (Lamequat^{®}L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine^{®}/Sandoz), Co-polymere der Acrylsäure mit Dimethyldiallyl-ammoniumchlorid (Merquat^{®} 550/Chemviron), Polyaminopolyamide, wie z. B. beschrieben in der FR-A 2252840 sowie deren vernetzte wasserlöslichen Polymere, Kondensationsprodukte aus Dihalogenalkylen, wie z. B. Dibrombutan mit Bisdialkylaminen, wie z. B. Bis-Dimethylamino-1,3-propan, kationischer Guar-Gum, wie z. B. Jaguar^{®} CBS, Jaguar^{®} C-17, Jaguar^{®} C-16 der Firma Celanese, quaternierte Ammoniumsalz-Polymere, wie z.B. Mirapol^{®} A-15, Mirapol^{®} AD-1, Mirapol^{®} AZ-1 der Firma Miranol.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von 5 ·105 bis 5 · 10⁶ (g/mol) auf.

Zur Herstellung erfindungsgemäßer Zubereitungen muss das Chitosan in die Salzform überführt werden. Dies kann durch Auflösen in verdünnten wässrigen Säuren erfolgen. Als Säuren sind sowohl Mineralsäuren wie z. B. Salzsäure, Schwefelsäure und Phosphorsäure als auch organische Säuren, z. B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z. B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z. B. Glycolsäure oder Milchsäure verwendet.

Die kationischen Polymere werden in den erfindungsgemäßen Zusammensetzungen bevorzugt in einer Menge von 0,1 bis 15 Gew.-%, insbesondere in einer Menge von 0,2 bis 5 Gew.-%, jeweils bezogen auf das Gesamtgewicht der Zusammensetzungen, eingesetzt.

Als kationische Tenside können erfindungsgemäß bevorzugt Tenside vom Typ der quartären Ammoniumverbindungen, der Esterquats und der Amidoamine eingesetzt werden. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27, Quaternium-87, Behentrimonium Chloride und Quaternium-83 bekannten Imidazolium-Verbindungen. Die langen Alkylketten der oben genannten Tenside weisen bevorzugt 10 bis 22 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substänzgruppe stellt das unter der Bezeichnung Tegoamid^{®} S 18 im Handel erhältliche Stearamidopropyldimethylamin dar.

In einer bevorzugten Ausführungsform der Erfindung können als kationische Tenside Verbindungen der Formel (II) eingesetzt werden, 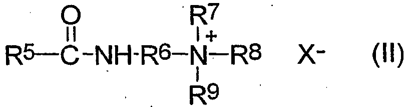 in denen R⁵ steht für eine gesättigte oder ungesättigte, lineare, verzweigte oder cyclische Alkyl- oder Arylgruppe mit 8 bis 24 C-Atomen; R⁶ steht für eine gesättigte oder ungesättigte, lineare, verzweigte oder cyclische, substituierte oder nicht substituierte Alkylengruppe mit 1 bis 10 C-Atomen und 0 bis 5 Hydroxygruppen; R⁷, R⁸, R⁹ stehen unabhängig voneinander für Wasserstoff, eine C₁-C₄-Alkylgruppe, eine C₅- oder C₆-Cycloalkylgruppe, eine Arylgruppe oder eine C₂-C₄-Hydroxyalkylgruppe, und X⁻ steht für ein monovalentes Anion.

Erfindungsgemäß bevorzugte Verbindungen nach Formel (II) weisen für den Rest R⁵ eine gesättigte, lineare C-₁₀-C₁₈-Alkylgruppe; für den Rest R⁶ eine Gruppe CH₂CH₂CH₂-; für die Reste R⁷, R⁸ und R⁹ jeweils Allylgruppen, beispielsweise Methyl-, Ethyl-, Propyl-, Isopropyl- und Butylgruppen, insbesondere bevorzugt Methylgruppen und für das monovalente Anion X⁻ Halogenid, beispielswiese Chlorid oder Bromid oder eine der beiden Gruppen CH₃-O-SO₃⁻ (Methosulfat) oder CH₃CH₂-O-SO₃⁻ (Ethosulfat), auf.

Insbesondere bevorzugte Verbindungen nach Formel (II) sind im Sinne der Erfindung Palmitamidopropyltrimonium Chloride (beispielsweise unter den Handelsnamen Varisoft^{®} PATC und/oder Varisoft^{®} Clear (INCI-Bezeichnung: Palmitamidopropyltrimonium Chloride and Trimethyl Pentanol Hydroxyethyl Ether and PPG-3 Myristyl Ether) von der Firma Degussa), Behenamidopropyl Ethyldimonium Ethosulfate (beispielsweise Mackernium^{®} BAPDES der Firma McIntyre), Stearamidopropyl Trimonium Methosulfate (beispielsweise Catigene^{®} SA-70 der Firma Stepan Company) und Undecylamidopropyltrimonium Methosulfate (beispielsweise Rewocid^{®} UTM 185 der Firma Degussa).

Ein ganz besonders bevorzugtes kationisches Tensid im Sinne der Erfindung ist das unter dem Handelsnamen Varisoft^{®} Clear oder Varisoft^{®} PATC vertriebene Palmitamidopropyltrimonium Chloride.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind bevorzugt; Mengen von 0,1 bis 2 Gew.-% sind besonders bevorzugt.

In einer sechsten, besonders bevorzugten Ausführungsform der Erfindung, enthalten die Haarbehandlungsmittel zur weiteren Unterstützung der haarfixierenden Eigenschaften Polymere. Diese sind üblicherweise ausgewählt aus amphoteren und nichtionischen Polymeren.

Unter amphoteren Polymeren im Sinne der Erfindung werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die-COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel (III),

   R¹⁰-CH=CR¹¹-CO-Z-(CₙH₂ₙ)-N⁽⁺⁾R¹²R¹³R¹⁴ A⁽⁻⁾ (III)

   in der R¹⁰ und R¹¹ unabhängig voneinander stehen für Wasserstoff oder eine Methylgruppe und R¹², R¹³ und R¹⁴ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
(b) monomeren Carbonsäuren der allgemeinen Formel (IV),

   R¹⁵-CH=CR¹⁶-COOH (IV)

   in denen R¹⁵ und R¹⁶ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R¹², R¹³ und R¹⁴ Methylgruppen sind, Z eine NH-Gruppe und A⁽⁻⁾ ein Halogenid-, Methoxysulfat- oder Ethoxysulfat-Ion ist; Acrylamidopropyl-trimethylammoniumchlorid ist ein besonders bevorzugtes Monomeres (a). Als Monomeres (b) für die genannten Polymerisate wird bevorzugt Acrylsäure verwendet.

Die erfindungsgemäßen Mittel können in einer dritten Variante weiterhin nichtionogene Polymere enthalten.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®}. VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) vertrieben werden.
- Synthetische Polymere z.B. Aristoflex AVC
- Schellack
- Polyvinylpyrrolidone, wie sie beispielsweise unter der Bezeichnung Luviskol^{®} (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder HydroxyGruppen enthalten.
- Glycosidisch substituierte Silicone gemäß der EP 0612759 B1.

Es ist erfindungsgemäß auch möglich, daß die verwendeten Zubereitungen mehrere, insbesondere zwei verschiedene Polymere gleicher Ladung und/oder jeweils ein ionisches und ein amphoteres und/oder nicht ionisches Polymer enthalten.

Unter dem Begriff Polymer sind erfindungsgemäß ebenfalls spezielle Zubereitungen von Polymeren wie sphärische Polymerpulver zu verstehen. Es sind verschiedene Verfahren bekannt, solche Mikrokugeln aus verschiedenen Monomeren herzustellen, z.B. durch spezielle Polymerisationsverfahren oder durch Auflösen des Polymeren in einem Lösungsmittel und Versprühen in ein Medium, in dem das Lösungsmittel verdunsten oder aus den Teilchen herausdiffundieren kann. Ein solches Verfahren ist z.B. aus EP 466 986 B1 bekannt. Geeignete Polymerisate sind z.B. Polycarbonate, Polyurethane, Polyacrylate, Polyolefine, Polyester oder Polyamide. Besonders geeignet sind solche sphärischen Polymerpulver, deren Primärpartikeldurchmesser unter 1 µm liegt. Solche Produkte auf Basis eines Polymethacrylat-Copolymers sind z.B. unter dem Warenzeichen Polytrap^{®}Q5-6603 (Dow Corning) im Handel. Andere Polymerpulver, z.B. auf Basis von Polyamiden (Nylon 6, Nylon 12) sind mit einer Teilchengröße von 2 - 10 µm (90 %) und einer spezifischen Oberfläche von ca. 10 m²/g unter der Handelsbezeichnung Orgasol^{®} 2002 DU Nat Cos (Atochem S.A., Paris) erhältlich. Weitere sphärische Polymerpulver, die für den erfindungsgemäßen Zweck geeignet sind, sind z.B. die Polymethacrylate (Micropearl M) von SEPPIC oder (Plastic Powder A) von NIKKOL, die Styrol-Divinylbenzol-Copolymeren (Plastic Powder FP) von NIKKOL, die Polyethylen- und Polypropylen-Pulver (ACCUREL EP 400) von AKZO, oder auch Silikonpolymere (Silicone Powder X2-1605) von Dow Corning oder auch sphärische Cellulosepulver.

Die weiteren Polymere sind in den erfindungsgemäß verwendeten Mitteln bevorzugt in Mengen von 0,01 bis 10 Gew.-%, bezogen auf das gesamte Mittel, enthalten. Mengen von 0,1 bis 5, insbesondere von 0,1 bis 3 Gew.-%, sind besonders bevorzugt.

In einer siebten, besonders bevorzugten Ausführungsform der Erfindung, enthalten die Haarbehandlungsmittel weiterhin mindestens ein Silikon. Erfindungsgemäß geeignete Silikone sind insbesondere Dialkyl- und Alkylarylsiloxane, wie beispielsweise Dimethylpolysiloxan und Methylphenylpolysiloxan, sowie deren alkoxylierte und quaternierte Analoga.

Weitere Beispiele für erfindungsgemäß geeignete Silikone sind:
- Silikon-Glykol-Copolymere (INCI-Bezeichnung: Dimethicone Copolyol), z. B. die Handelsprodukte DC 190 und DC 193 von Dow Corning,
- aminofunktionelle Polydimethylsiloxane und hydroxyaminomodifizierte Silikone (INCI-Bezeichnung: u.a. Amodimethicone und Quaternium-80), wie die Handelsprodukte Dow Corning^{®} 929 Emulsion, Dow Corning^{®} 939 Emulsion, Dow Corning^{®} 949 Emulsion ,SM-2059 (Hersteller: General Electric), SLM-55067 (Hersteller: Wacker) sowie Abil^{®}-Quat 3270 und 3272 (Hersteller: Goldschmidt).

Die erfindungsgemäßen Haarbehandlungsmittel enthalten die Silikone bevorzugt in Mengen von 0,05 bis 10 Gew.-%, bezogen auf das Gesamtgewicht des Haarbehandlungsmittels.

Neben der erfindungsgemäß bevorzugten, sprühbaren Konfektionierungsform des Haarbehandlungsmittels sind auch weitere Konfektionierungsformen des Haarbehandlungsmittels möglich. Üblicherweise haben die Haarbehandlungsmittel eine wässrige oder wässrig-alkoholische Basis. Als Alkohole kommen dabei insbesondere niedere Alkohole wie Ethanol und Isopropanol in Betracht, Wässrig-alkoholische Grundlagen können dabei Wasser-Alkohol, bevorzugt in einem Verhältnis von 1 : 5 bis 5 : 1, enthalten. Eine bevorzugte wässrig-alkoholische Basis weist einen Alkoholgehalt von bis zu 15 Gew.-%, bezogen auf die Wassermenge, auf.

Die erfindungsgemäßen Haarbehandlungsmittel können sowohl auf dem Haar verbleiben, als auch nach einer Einwirkungszeit, die mehrere Minuten bis zu etwa einer Stunde beträgt, wieder ausgespült werden. Auf dem Haar verbleibende erfindungsgemäße Haarbehandlungsmittel haben sich als besonders wirksam erwiesen und können daher eine besonders bevorzugte Ausführungsformen der erfindungsgemäßen Lehre darstellen.

Gemäß einer weiteren bevorzugten Ausführungsform werden die erfindungsgemäßen, sprühbaren Haarbehandlungsmittel als Haarkur, Haar-Conditioner, Haarspitzen-Fluid oder Haarwasser formuliert. Die erfindungsgemäßen Haarbehandlungsmittel dieser Ausführungsform können nach Ablauf der Einwirkungszeit wieder mit Wasser ausgespült werden; bevorzugt werden sie jedoch auf dem Haar belassen. In einer ganz besonders bevorzugten Ausführungsform werden die erfindungsgemäßen Haarkuren und Haar-Conditioner in einer sprühbaren Variante formuliert. Dazu können die Mittel weiterhin Treibgase enthalten; bevorzugt in dieser Variante ist jedoch die Formulierung als Pumpspray mit Luft als Treibmittel.

Gemäß einer weiteren bevorzugten Ausführungsform kann es sich bei den erfindungsgemäßen, sprühbaren Mitteln um festigende Mittel wie Haarfestiger, Schaumfestiger, Haarsprays, Sprühfestiger, Styling-Gels und Fönwellmitteln handeln. Entsprechend können die Haarbehandlungsmittel als Lösungen, Emulsionen, Gele, Cremes, Aerosole oder Lotionen formuliert werden. Sofern diese Mittel Komponenten enthalten, die miteinander in den Haarbehandlungsmittel über längere Zeit nicht lagerstabil sind, so ist es auch möglich, die erfindungsgemäßen Haarbehandlungsmittel in Form von zwei oder mehr Teil-Zubereitungen lagerstabil zu formulieren. Die zur Anwendung bestimmten Häarbehandlungsmittel können dann unmittelbar vor der Anwendung durch Mischung der Teil-Zubereitungen hergestellt werden.

Der pH-Wert der erfindungsgemäßen Haarbehandlungsmittel kann prinzipiell bei Werten von 2 bis 7 liegen. Er liegt bevorzugt zwischen 2 und 6, wobei Werte von 3 bis 5 besonders bevorzugt sind. Zur Einstellung dieses pH-Wertes kann praktisch jede für kosmetische Zwecke einsetzbare Säure verwendet werden. Üblicherweise werden als Säuren Genussäuren verwendet. Unter Genussäuren werden solche Säuren verstanden, die im Rahmen der üblichen Nahrungsaufnahme aufgenommen werden und positive Auswirkungen auf den menschlichen Organismus haben. Genussäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt.

Neben den erfindungsgemäß zwingend erforderlichen Bestandteilen können die erfindungsgemäßen Haarbehandlungsmittel weiterhin einen oder mehrere der folgenden Bestandteile enthalten:
- Entschäumer wie Silikone,
- Verdickungsmittel wie Agar-Agar, Guar-Gum, Alginate, Xanthan-Gum, Gelatine, Pektine, Hydroxyethylcellulose sowie Polyacrylamide und deren Copolymere,
- Strukturanten wie Maleinsäure,
- Parfümöle, Dimethylisosorbit und Cyclodextrine,
- Lösungsvermittler wie Ethanol, Isopropanol, Ethylenglykol, Propylenglykol, Glycerin und Diethylenglykol,
- Farbstoffe zum Anfärben des Mittels,
- Antischuppenwirkstoffe wie Piroctone Olamine, Zink Omadine und Climbazol,
- Weitere Substanzen zur Einstellung des pH-Wertes,
- Wirkstoffe wie Allantoin, Panthenol, Pantolacton, Nicotinsäureamid, Pyrrolidoncarbonsäuren, Vitamine und Bisabolol,
- Pflanzenextrakte, insbesondere aus Eichenrinde, Brennessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Schafgarbe, Hauhechel, Meristem, Ginseng und Ingwerwurzel,
- faserstrukturverbessernde Wirkstoffe, insbesondere Mono-, Di- und Oligosaccharide wie beispielsweise Glucose, Galactose, Fructose, Fruchtzucker und Lactose,
- konditionierende Wirkstoffe wie Paraffinöle, Polydecene, Squalene, pflanzliche Öle, z.B. Sonnenblumenöl, Orangenöl, Mandelöl, Weizenkeimöl und Pfirsichkernöl sowie Phospholipide, beispielsweise Sojalecithin und Kephaline,
- Lichtschutzmittel,
- Konsistenzgeber wie Zuckerester, Polyolester und Polyalkylether,
- Fettsäurealkanolamide,
- Quell- und Penetrationsstoffe wie Glycerin, Propylenglycolmonoethylether, Guanidine, Harnstoffe sowie primäre, sekundäre und tertiäre Phosphate,
- Trübungsmittel wie Latex, Styrol/PVP- und Styrol/Acrylamid-Copolymere,
- Perlglanzmittel wie Ethylenglykolmono- und -distearat,
- Komplexbildner wie EDTA, NTA, β-Alanindiessigsäure und Phosphonsäuren,
- Treibmittel wie Propan-Butan-Gemische, N₂O, Dimethylether, CO₂, N₂ und Luft sowie
- Antioxidantien.

Ein weiterer Gegenstand der Erfindung ist die Verwendung des erfindungsgemäßen Haarbehandluhgsmittels zum Styling der Haare und zur Haarfixierung.

Ein weiterer Gegenstand der Erfindung ist die Verwendung des erfindungsgemäßen Haarbehandlungsmittels zur Pflege von Haaren.

Ein weiterer Gegenstand der Erfindung ist ein Verfahren zur Behandlung von Haaren, bei dem ein erfindungsgemäßes Haarbehandlungsmittel auf die Haare aufgebracht und dort belassen wird.

Die folgenden Beispiele sollen den Gegenstand der Anmeldung näher beschreiben, ohne ihn darauf zu beschränken. Die Mengenangaben beziehen sich, sofern nicht anders angegeben, auf Gew.-% in der gesamten Zusammensetzung.

### Beispiele

**Beispiel 1:**

| | |
|---|---|
| ENTSALZTES WASSER | 89,279940 |
| NATRIUMBENZOAT | 0,100000 |
| PANTHENOL 75 L | 0,500000 |
| DOW CORNING^{®} 193 ⁽¹⁾ | 0,400000 |
| DEHYQUART^{®} A-CA 25 % ⁽²⁾ | 0,400000 |
| TIMIRON SUPER BLUE | 0,010000 |
| STRUCTURE^{®} PLUS ⁽³⁾ | 4,000000 |
| SYNTHALEN^{®} CR ⁽⁴⁾ | 0,300000 |
| VARISOFT^{®} CLEAR ⁽⁵⁾ | 0,200000 |
| TIMIRON ARCTIC SILVER | 0,010000 |
| RED 33 | 0,000060 |
| CREMOPHOR^{®} CO 40 ⁽⁶⁾ | 0,300000 |
| PARFUM | 0,200000 |
| MILCHSÄURE 80 % | 0,300000 |
| WEIZENPROTEINHYDROLYSAT KATIONISIERT | 1,000000 |
| PROMOIS^{®} SILK 1000 ⁽⁷⁾ | 1,000000 |
| HYDAGEN^{®} HCMS-LA (3%ige Lsg.)⁽⁸⁾ | 2,000000 |

**Beispiel 2:**

| | |
|---|---|
| ENTSALZTES WASSER | 81,669940 |
| PANTHENOL 75 L | 0,300000 |
| DOW CORNING^{®} 193 ⁽¹⁾ | 0,200000 |
| DEHYQUART^{®} A-CA 25 % ⁽²⁾ | 0,400000 |
| TIMIRON SUPER BLUE | 0,010000 |
| STRUCTURE^{®} PLUS ⁽³⁾ | 3,500000 |
| SYNTHALEN^{®} CR ⁽⁴⁾ | 0,250000 |
| VARISOFT^{®} CLEAR ⁽⁵⁾ | 0,400000 |
| TIMIRON ARCTIC SILVER | 0,010000 |
| TIMIRON SUPER BLUE | 0,010000 |
| RED 33 | 0,000060 |
| CREMOPHOR^{®} CO 40 ⁽⁶⁾ | 0,300000 |
| PARFUM | 0,350000 |
| MILCHSÄURE 80 % | 0,800000 |
| WEIZENPROTEINHYDROLYSAT KATIONISIERT | 1,000000 |
| PROMOIS^{®} SILK 1000 ⁽⁷⁾ | 1,000000 |
| SERICIN^{®(9)} | 0,200000 |
| ETHYLALKOHOL VERGAELLT 96 VOL % FLS | 10,000000 |

**Beispiel 3:**

| | |
|---|---|
| ENTSALZTES WASSER | 81,669940 |
| NATRIUMBENZOAT | 0,100000 |
| PANTOLACTONE^{® (10)} | 0,500000 |
| DOW CORNING^{®} 193 ⁽¹⁾ | 0,500000 |
| DEHYQUART^{®} A-CA 25 % ⁽²⁾ | 0,400000 |
| STRUCTURE^{®} PLUS ⁽³⁾ | 5,000000 |
| SYNTHALEN^{®} CR ⁽⁴⁾ | 0,200000 |
| ARISTOFLEX^{®} AVC ⁽¹¹⁾ | 0,400000 |
| EUPERLAN^{®} PK 1200 ⁽¹²⁾ | 2,000000 |
| CREMOPHOR^{®} CO 40 ⁽⁶⁾ | 0,300000 |
| PARFUM | 0,250000 |
| MILCHSÄURE 80 % | 0,250000 |
| WEIZENPROTEINHYDROLYSAT KATIONISIERT | 0,500000 |
| HYDAGEN^{®} HCMS-LA (3%ige Lsg.) ⁽⁸⁾ | 2,000000 |

| | |
|---|---|
| ⁽¹⁾ Silicon Glykol Copolymer (INCI-Bezeichnung: PEG-12 Dimethicone; Dow Corning) ⁽²⁾ Trimethylhexadecylammoniumchlorid (INCI-Bezeichnung: Cetrimonium Chloride; Cognis Deutschland GmbH) ⁽³⁾ Structure plus (INCI-Bezeichnung: Acrylates/Aminoacrylates/C10-30 Akryl PEG 20 Itaconate Copolymer; National Starch) ⁽⁴⁾ Homopolymer *N,N,N-Poly(cholinchloridmethacrylat) (INCI-Bezeichnung: Polyquaternium-37; 3V Sigma) ⁽⁵⁾ Varisoft Clear (INCI-Bezeichnung: Palmitamidopropyltrimonium Chloride, PPG-3 Myristyl Ether, Trimethyl Pentanol Hydroxyethylether; Degussa) ⁽⁶⁾ Cremophor CO 40 (INCI-Bezeichnung: PEG-40 Hydrogenated Castor Oil; BASF) ⁽⁷⁾ Collagen Hydrolysat (INCI-Bezeichnung: Hydrolyzed Silk; RITA Corporation) ⁽⁸⁾ Chitosanlactat (INCI-Bezeichnung: Chitosan Lactate; Cognis) ⁽⁹⁾ Seidenproteinhydrolysat (INCI-Bezeichnung: Sericin; Pentapharm) ⁽¹⁰⁾ Hydroxy-3,3-dimethyl-.gamma.-butyrolacton *D,L-2- (INCI-Bezeichnung: Pantolactone; Dow) ⁽¹¹⁾ Polymer Sulfonsäure-Ammoniumsalz (INCI-Bezeichnung: Ammonium Acryloyldimethyltaurate/VP Copolymer; Clariant) ⁽¹²⁾ Euperlan PK 1200 (INCI-Bezeichnung: Coco-Glusoside, Glycol Distearate, Glycerin; Cognis Deutschland GmbH) | |

## Patentansprüche

1. Klares, sprühbares Haarbehandlungsmittel in dem Pigmente schweben, enthaltend in einem kosmetisch akzeptablen Träger
a) 0,001 bis 15 Gew.-% mindestens eines von b) verschiedenen, verdickend wirkenden Polymers und
b) 0,001 bis 15 Gew.-% mindestens eines Acrylterpolymers, das zusammengesetzt ist aus
- 5 bis 80% eines Acrylmonomers A, das ausgewählt ist aus C₁-C₆-Alkylacrylaten und C₁-C₈-Alkylmethacrylaten,
5 bis 80% eines Monomers B, das ausgewählt ist aus Vinyl-substituierten Heterocyclen, die mindestens ein Stickstoff- oder Schwefelatom enthalten, (Meth)acrylamid, Mono- oder Di-C₁₋₄-alkyl(meth)arcylaten und Mono- oder Di-C₁₋₄-alkylamino-C₁₋₄-alkyl-(meth)-acrylamiden
wobei das Monomer B N,N-Dimethylylaminoethylmethacrylat, N,N-Diethylaminoethylacrylat, N,N-Diethylaminoethylmethacrylat, N,t-Butylaminoethylacrylat, N,t-Butylaminoethylmethacrylat, N,N-Dimethylaminopropylacrylamid, N,N-Dimethylaminopropylmethacrylamid, N,N-Diethylaminopropylacrylamid oder N,N-Diethylaminopropylmethacrylamid ist, und
- 0,1 bis 30% eines Monomers C, das ausgewählt ist aus
- einem ethylenisch ungesättigten copolymerisierbaren oberflächenaktiven Monomer, erhältlich durch Kondensation eines nicht-ionischen Tensids mit einer α,β-ethylenisch ungesättigten Carbonsäure oder dem Anhydrid davon und
- einem oberflächenaktiven Monomer, ausgewählt aus dem Harnstoff-Reaktionsprodukt eines monoethylenisch ungesättigten Monoisocyanats mit einem nicht-ionischen Tensid mit Amin-Funktionalität,
**dadurch gekennzeichnet, dass** es eine Viskosität von 100 bis 600 mPas aufweist gemessen bei 20°C mit Brookfield RVDVII- Viskosimeter bei 20 UpM, Spindel 3).

2. Haarbehandlungsmittel nach Anspruch 1, **dadurch gekennzeichnet, dass** das verdickende Polymer a) ausgewählt ist aus Homopolymeren der Formel (I), 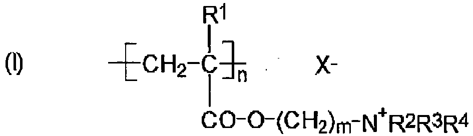 in der R¹ für Wasserstoff oder eine Methylgruppe steht, R², R³ und R⁴ unabhängig voneinander für C₁₋₄-Alkyl-, -Alkenyl- oder -Hydroxyalkylgruppen stehen; m für Zahlen von 1 bis 4 steht, n für eine natürliche Zahl und X⁻ für ein physiologisch verträgliches organisches oder anorganisches Anion steht, sowie Copolymeren, bestehend im wesentlichen aus den in Formel (I) aufgeführten Monomereneinheiten sowie nichtionogenen Monomereneinheiten.

3. Haarbehandlungsmittel nach einem der Ansprüche 1 oder 2, **dadurch gekennzeichnet, dass** die Reste R¹ bis R⁴ für Methylgruppen stehen und m den Wert 2 hat

4. Haarbehandlungsmittel nach einem der Ansprüche 1 bis 3, **dadurch gekennzeichnet, dass** das verdickend wirkende Polymer a) ein Homopolymer nach Formel (I) ist.

5. Haarbehandlungsmittel nach einem der Ansprüche 1 bis 3, **dadurch gekennzeichnet, dass** das Polymer a) ein Copolymer aus Monomereneinheiten der Formel (I) und nichtionogenen Monomereneinheiten ist, dessen nichtionogene Monomereneinheiten ausgewahlt sind aus der Gruppe, die von Acrylamid, Methacrylamid, Acrylsäure-C₁₋₄-Alkylestern, Methacrylsäure-C₁₋₄-Alkylestern und Vinylpyrrolidon gebildet wird.

6. Haarbehandlungsmittel nach Anspruch 5, **dadurch gekennzeichnet, dass** die nichtionogene Einhelt Acrylam id ist.

7. Haarbehandlungsmittel nach Anspruch 5, **dadurch gekennzeichnet, dass** die nichtionogene Einheit Vinylpyrrolidon ist.

8. Haarbehandlungsmittel nach einem der Ansprüche 1 bis 7, **dadurch gekennzeichnet, dass** das Monomer A ein C₂₋₆-Alkylacrylat ist.

9. Haarbehandlungsmittel nach einem der Ansprüche 1 bis 8, **dadurch gekennzeichnet, dass** das Monomer C ein copolymerisierbares ethylenisch ungesättigtes Tensidmonomer ist, das durch Kondensation eines nichtionischen grenzflächenaktiven Stoffes mit Itaconsäure entsteht.

10. Haarbehandlungsmittel nach einem der Ansprüche 1 bis 9, **dadurch gekennzeichnet, dass** es weiterhin mindestens ein Proteinhydrolysat und/oder mindestens eine kationische Konditionierkomponente, ausgewählt aus kationischen Tensiden und/oder kationischen Polymeren, und/oder mindestens eine Silikonkomponente enthält.

11. Verwendung eines Haarbehandlungsmittels nach einem der Ansprüche 1 bis 10 zum Styling, zur Haarfixierung und zur Pflege von Haaren.

12. Verfahren zur Behandlung von Haaren, **dadurch gekennzeichnet, dass** man eine Zubereitung gemäß einem der Ansprüche 1 bis 10 auf die Haare aufbringt und sie darauf belässt.

## Claims

1. A clear, sprayable hair treatment agent in which pigments are suspended, containing in a cosmetically acceptable carrier
a) from 0.001 to 15 % by weight of at least one polymer that has a thickening action and is different from b)
b) from 0.001 to 15 % by weight of at least one acrylic terpolymer composed of
- from 5 to 80 % of an acrylic monomer A selected from C₁-C₆ alkyl acrylates and C₁-C₆ alkyl methacrylates,
from 5 to 80 % of a monomer B selected from vinyl-substituted heterocycles containing at least one nitrogen or sulfur atom, (meth)acrylamide, mono- or di-C₁₋₄-alkyl(meth)acrylates and mono- or di-C₁₋₄-alkylamino-C₁₋₄-alkyl-(meth)-acrylamides,
monomer B being N,N-dimethylaminoethyl methacrylate, N,N-diethylaminoethyl acrylate, N,N-diethylaminoethyl methacrylate, N,t-butylaminoethyl acrylate, N,t-butylaminoethyl methacrylate, N,N-dimethylaminopropyl acrylamide, N,N-dimethylaminopropyl methacrylamide, N,N-diethylaminopropyl acrylamide or N, N-diethylaminopropyl methacrylamide, and
- from 0.1 to 30 % of a monomer C selected from
- an ethylenically unsaturated, copolymerizable surface-active monomer obtainable by condensing a non-ionic surfactant with an α,β-ethylenically unsaturated carboxylic acid or the anhydride thereof, and
- a surface-active monomer selected from the urea reaction product of a monoethylenically unsaturated monoisocyanate and a non-ionic surfactant having an amine functionality,
**characterized in that** said agent has a viscosity of from 100 to 600 mPa, measured at 20 °C using a Brookfield RVDVII+ viscometer at 20 UpM with spindle 3).

2. The hair treatment agent according to claim 1, **characterized in that** the thickening polymer a) is selected from homopolymers of formula (I), 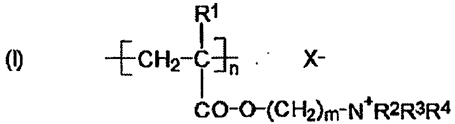 in which R¹ represents hydrogen or a methyl group, R², R³ and R⁴ represent independently of one another C₁₋₄ alkyl, alkenyl or hydroxyalkyl groups, m represents numbers from 1 to 4, n represents a natural number and X⁻ represents a physiologically acceptable organic or inorganic anion, and copolymers substantially consisting of the monomer units specified in formula (I) and non-ionogenic monomer units.

3. The hair treatment agent according to one of claims 1 or 2, **characterized in that** the functional groups R¹ to R⁴ represent methyl groups and m has the value 2.

4. The hair treatment agent according to one of claims 1 to 3, **characterized in that** polymer a), which has a thickening action, is a homopolymer according to formula (I).

5. The hair treatment agent according to one of claims 1 to 3, **characterized in that** polymer a) is a copolymer consisting of monomer units of formula (I) and non-ionogenic monomer units, the non-ionogenic monomer units of which are selected from the group formed of acrylamide, methacrylamide, acrylic acid C₁₋₄ alkyl esters, methacrylic acid C₁₋₄ alkyl esters and vinylpyrrolidone.

6. The hair treatment agent according to claim 5, **characterized in that** the non-ionogenic unit is acrylamide.

7. The hair treatment agent according to claim 5, **characterized in that** the non-ionogenic unit is vinyl pyrrolidone.

8. The hair treatment agent according to one of claims 1 to 7, **characterized in that** monomer A is a C₂₋₆ alkyl acrylate.

9. The hair treatment agent according to one of claims 1 to 8, **characterized in that** monomer C is a copolymerizable, ethylenically unsaturated surfactant monomer which is formed by condensing a non-ionic interfacial-active substance with itaconic acid.

10. The hair treatment agent according to one of claims 1 to 9, **characterized in that** it also contains at least one protein hydrolyzate and/or at least one cationic conditioning component selected from cationic surfactants and/or cationic polymers, and/or at least one silicon component.

11. The use of a hair treatment agent according to one of claims 1 to 10 for styling, for retaining hairstyles and for caring for hair.

12. A method for treating hair, **characterized in that** a preparation according to one of claims 1 to 10 is applied to the hair and left thereon.

## Revendications

1. Produit de traitement capillaire transparent pulvérisable, dans lequel flottent des pigments, contenant dans un vecteur cosmétiquement acceptable :
a) de 0,001 % à 15 % en poids d'au moins un parmi différents polymères épaississants, et
b) de 0,001 % à 15 % en poids d'au moins un terpolymère acrylique constitué de :
- 5 à 80 % d'un monomère acrylique A choisi parmi des acrylates de C₁₋₆-alkyle et des méthacrylates de C₁₋₆-alkyle,
- 5 à 80 % d'un monomère B choisi parmi des hétérocycles vinyl-substitué contenant au moins un atome d'azote ou de soufre, un (méth)acrylamide, des mono- ou di-C₁₋₄-alkyl-(méth)acrylates et des mono- ou di-C₁₋₄-alkylamino- C₁₋₄-alkyl-(méth)acrylamides,
le monomère B étant N,N-diméthylaminoéthylméthacrylate, N,N-diéthylaminoéthylacrylate, N,N-diéthylaminoéthylméthacrylate, N-tertiobutylaminoéthylacrylate, N-tertiobutylaminoéthylméthacrylate, N,N-diméthylaminopropylacrylamide, N,N-diméthylaminopropylméthacrylamide, N,N-diéthylaminopropylacrylamide ou N,N-diéthylaminopropylméthacrylamide, et
- 0,1 % à 30 % d'un monomère C choisi parmi :
- un monomère insaturé éthyléniquement, copolymérisable et tensioactif, disponible par condensation d'un tensioactif non-ionique avec un acide carboxylique insaturé α-β-éthylénique ou son anhydride, et
- un monomère tensioactif choisi parmi le produit de réaction à l'urée d'un monoisocyanate insaturé monoéthylénique avec un tensioactif non-ionique avec une fonctionnalité amine,
**caractérisé en ce qu'**il présente une viscosité de 100 à 600 mPa.s mesurée à 20°C par un viscosimètre Brookfield RVDVII+ à 20 tr/min, cylindre 3.

2. Produit de traitement capillaire selon la revendication 1, **caractérisé en ce que** le polymère épaississant a) est choisi parmi des homopolymères de formule (I) : 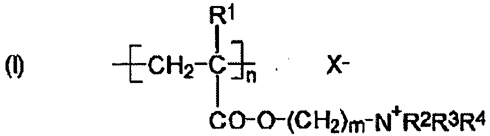 où R¹ représente un hydrogène ou un groupe méthyle, R², R³ et R⁴ représentent, indépendamment les uns des autres, des groupes C₁₋₄-alkyles, alcényles ou hydroxyalkyles, m représente des entiers de 1 à 4, n représente un entier naturel et X⁻ représente un anion organique ou minéral physiologiquement compatible, ainsi que parmi des copolymères constitués essentiellement d'unités monomères illustrées par la formule (I) ainsi que d'unités monomères non-ionogènes.

3. Produit de traitement capillaire selon une des revendications 1 ou 2, **caractérisé en ce que** les résidus R¹ à R⁴ représentent des groupes méthyles et m = 2.

4. Produit de traitement capillaire selon une des revendications 1 ou 3, **caractérisé en ce que** le polymère épaississant a) est un homopolymère selon la formule (I).

5. Produit de traitement capillaire selon une des revendications 1 ou 3, **caractérisé en ce que** le polymère épaississant a) est un copolymère constitué d'unités monomères selon la formule (I) et d'unités monomères non-ionogènes, dont les unités monomères non-ionogènes sont choisies parmi le groupe formé de l'acrylamide, le méthacrylamide, les acrylates de C₁₋₄-alkyles, les méthacrylates de C₁₋₄-alkyles et la vinylpyrrolidone.

6. Produit de traitement capillaire selon la revendication 5, **caractérisé en ce que** l'unité non-ionogène est un acrylamide.

7. Produit de traitement capillaire selon la revendication 5, **caractérisé en ce que** l'unité non-ionogène est une vinylpyrrolidone.

8. Produit de traitement capillaire selon une des revendications 1 à 7, **caractérisé en ce que** le monomère A est un C₂₋₆-alkylacrylate.

9. Produit de traitement capillaire selon une des revendications 1 à 8, **caractérisé en ce que** le monomère C est un monomère tensioactif éthyléniquement insaturé formé par condensation d'une substance tensioactive non-ionique avec l'acide itaconique.

10. Produit de traitement capillaire selon une des revendications 1 à 9, **caractérisé en ce qu'**il contient en outre au moins un hydrolysat de protéine et/ou au moins un composant de conditionnement cationique choisi parmi des tensioactifs cationiques et/ou des polymères cationiques et/ou au moins un composant silicone.

11. Utilisation d'un produit de traitement capillaire selon une des revendications 1 à 10 pour la coiffure, la fixation des cheveux et le soin des cheveux.

12. Procédé de traitement capillaire **caractérisé en ce qu'**on applique une préparation selon une des revendications 1 à 10 sur les cheveux et qu'on la laisse en place.
